# EUROPEAN PATENT APPLICATION

(11) **EP 2 601 974 A1**
(43) Date of publication of application: **12.06.2013**
(21) Application number: 11814693.5
(22) Date of filing: 04.08.2011
(51) Int. Cl.: A61K 47/34, A61K 9/70, A61K 47/02, A61K 47/10, A61K 47/12, A61K 47/18, A61K 47/22, A61K 47/26, A61K 47/28, A61K 47/32, A61K 47/38

(54) **PHARMACEUTICAL PREPARATION FOR CONTROLLING WATER-SOLUBLE DRUG RELEASE**

(30) Priority: 06.08.2010 JP 2010177252
(71) Applicant: Dainippon Sumitomo Pharma Co., Ltd., Osaka 541-8524 (JP)
(72) Inventor: MAEDA, Miho, Suita-shi Osaka 564-0053 (JP)
(74) Representative: Vossius & Partner
(86) International application number: PCT/JP2011/067837
(87) International publication number: WO 2012/018068

(57) **Abstract**

A solid formulation comprising a water-soluble drug and a hardly water-soluble pharmaceutically-acceptable solid substance, which comprises silicone as a carrier, wherein the silicone formulation can exhibit an excellent controlled-release of the water-soluble drug.

## Description

### TECHNICAL FIELD

The present invention relates to a parenteral sustained-release formulation comprising a silicone as a carrier, which is characterized by well controlling the release of a water-soluble drug *in vivo.*

### BACKGROUND ART

The sustained-release technology by controlling the release of a drug out of a drug formulation enables the supply of an active ingredient like continuous administrations, thereby the technology is expected to decrease the frequency of administration and also alleviate side effects because a high dose in one administration can be avoided.
In particular, as for drugs to be administered by injection, a lot of research on implantable sustained-release formulations comprising a polymer material as a carrier has been made. Amongst them, silicone, which is a synthetic polymer undegradable *in vivo*, has been used for a long time as a carrier in sustained-release formulations because it is biocompatible and has a good track record as a medical material for implant *in vivo*.

As an example of such sustained-release formulations comprising silicone as a carrier, Norplant^{™} is an implantable formulation wherein powdery levonorgestrel as an active ingredient is encapsulated in a cylindroid silicone container, which is characterized by continuing to release levonorgestrel *in vivo* for 5 years. And, as an example of matrix formulations, Compudose^{™} has a form wherein estradiol as an active ingredient is dispersed in silicone (*e.g*. Patent Reference 1).
Both the active ingredients in Norplant and Compudose are a lipophilic drug, which can be dissolved/diffused in silicone that is a hydrophobic polymer. Thereby, the drug on the surface of the formulation is spread to the surrounding tissues, and then the drug included in higher concentration inside the formulation is transferred by diffusion to the lower-concentrated surface of the formulation because the concentration of the lipophilic drug on the surface of the formulation decreases. Thus, the sustained release becomes possible.

On the other hand, water-soluble drugs are hardly dissolved in a hydrophobic polymer carrier and are unable to autonomously spread/release as opposed to lipophilic drugs. Thus, in order to release a water-soluble drug out of a hydrophobic polymer carrier, it is necessary to use a quite different release mechanism from that of a lipophilic drug.
One of general methods for releasing a water-soluble drug out of a hydrophobic polymer carrier is a release of the drug from pores of a reservoir-type formulation. Besides, there is another releasing mechanism wherein a drug is dispersed in a carrier, in which firstly a drug particle in close proximity to the surface of the formulation is eluted out by water in the surrounding tissues, and then another drug particle adjacent to the dissolved particle is eluted to the surface, that is, the phenomenon is sequentially repeated to form a continuing water channel system, and the drug is diffused in the channel while releasing the drug. Then, the difference in osmotic pressure produced inside the formulation can also make the inside of the formulation cracked to facilitate forming the channel and further can increase the release by the extruding effect of the swelling. Accordingly, it is necessary that each particle in a carrier lies adjacent to each other or the difference in osmotic pressure is produced inside the formulation, in order to continue the release. Thus, the mechanism is characterized by comprising more than a certain amount of a water-soluble drug or a water-soluble additive agent. As an example thereof, Patent Reference 2 discloses a method of controlling the release of a drug out of a silicone carrier by adding albumin.

However, such release system of a water-soluble drug is very difficult to control the release, *i.e*., in general, the initial release speed thereof is too fast and the drug is supposed to be explosively released, and then the release amount of the drug decreases over time like first-order release profile. After all, such release system is difficult to control the constant and steady release for a long time.
It is sometimes useful for a patient that the initial release speed is fast, but generally there are some problems, for example, side effects can happen due to such rapid increase of initial drug concentration, or the decrease of the drug release over time makes the use difficult. In particular, the initial release speed tends to be faster as the surface area is larger, hence, for a small formulation whose surface area is large per its weight, it is difficult to control the release while suppressing the initial burst. Thus, for such release system, it is difficult to try to miniaturize a formulation or reduce the thickness of a formulation for its purpose.
Patent Reference 3 discloses a technology that a water-soluble drug is sustainably released at a constant rate out of a hydrophobic polymer carrier, *i.e*., a columnar formulation wherein only the surround of a layer comprising a water-soluble drug is coated with an outer layer which can protect water and control the swelling of the inner layer. However, the technology has a disadvantage that it is impossible to miniaturize a formulation or formulate a thin film product. In addition, the drug-release surface thereof is only cross-section surfaces which are not coated with the outer layer, thereby the drug is localized around the cross-section surfaces in high concentration. Thus, the technology is not suitable for delivering a drug uniformly to the desired area.

Patent Reference 4 discloses a dressing that can release a drug sustainably, wherein a hydrophilic ingredient is used as an ingredient for controlling the drug release out of silicone carrier. The mechanism of releasing a drug is that firstly the hydrophilic ingredient becomes in hyperosmotic state, thereby the formulation is expanded, followed by the contraction of the silicone polymer to release the drug. However, such technology is substantially impracticable since the formulation can be expanded in an environment where water exists in the vicinity and then the volume of the formulation can be severely increased to compress the surrounding tissue when the formulation is used in vivo. Furthermore, the release speed of such formulation is fast, thus the release is expected to be only for several hours to several days, *i.e*., unsuitable for long-term sustained release. In addition, Patent Reference 4 teaches that preferred hydrophilic ingredients are liquid, and the most preferred hydrophilic ingredient is glycerol, and also exemplifies some liquid ingredients such as liquid polyethylene glycol, but these ingredients inhibit the shape-forming/curing of silicone, thus it is unsuitable to comprise such ingredients in the solid silicone formulations mentioned below.

As mentioned above, there were some trials to control the release of a water-soluble drug out of a silicone carrier, but there has not been any practicable formulation which can well control the release for a long time with little initial burst release, has a simple structure, can be easily prepared, can be transformed into various formulations such as rod shape and film shape according to application site, and can be suitably miniaturized, until now.
In addition, Patent Reference 5 discloses a sustained-release formulation for a lipophilic drug wherein a water-soluble substance is dispersed in a water-imperviable biocompatible material such as silicone.
Further, Patent Reference 6 discloses a transdermal formulation comprising a silicon-series or acrylic polymer as a base. In the basic polymer, a particle which is a microsphere encapsulated with a drug is dispersed, and the microsphere is made of a polymer such as crospovidone. The encapsulation of a drug in a microsphere brings in the effect for stabilizing a drug and the effect for promoting the transdermal absorption.

### PRIOR ART

### [Patent Reference]

- [Patent Reference 1]: JP 55-45694 A
- [Patent Reference 2]: JP 62-174007 A
- [Patent Reference 3]: JP 7-187994 A
- [Patent Reference 4]: JP 3-151322 A
- [Patent Reference 5]: WO 2000/015199
- [Patent Reference 6]: EP 0481443 A

### SUMMARY OF INVENTION

### (Problem to Be Solved by the Invention)

The purpose of the present invention is to provide a parenteral formulation which makes possible a good release-control of a water-soluble drug in a simply-structural matrix formulation without any complicated structure such as an outer coating for controlled-release, which is a practical silicone formulation that can be transformed into various formulations such as rod- and film-like shapes according to application site, and can be suitably miniaturized.

### (Means to Solve the Problem)

The present inventors have extensively studied to solve the above problem and then have found that it is possible to achieve a good controlled-release of a water-soluble drug out of a matrix-type silicone formulation by comprising a hardly water-soluble solid substance as an additive agent in the matrix formulation wherein the water-soluble drug is uniformly dispersed in the silicone. Based upon the new findings, the present invention has been completed.

The present invention provides inventions of various embodiments described below.
Term 1. A parenteral solid formulation comprising a water-soluble drug and a hardly water-soluble pharmaceutically-acceptable solid substance, which comprises silicone as a carrier.

Term 2. The parenteral solid formulation of Term 1 wherein the hardly water-soluble substance is low substituted hydroxypropylcellulose, partly pregelatinized starch, crospovidone, croscarmellose sodium, myristic acid, cholesterol and/or saccharin.
Term 3. The parenteral solid formulation of Term 1 wherein the hardly water-soluble substance is low substituted hydroxypropylcellulose and/or cholesterol.

Term 4. The parenteral solid formulation of any one of Terms 1 to 3 wherein the hardly water-soluble substance is contained in 3 to 35 % by weight per the whole weight of the formulation.

Term 5. The parenteral solid formulation of any one of Terms 1 to 4 wherein the silicone is contained in 55 % or more by weight per the whole weight of the formulation.

Term 6. The parenteral solid formulation of any one of Terms 1 to 5 further comprising a water-soluble additive agent.

Term 7. The parenteral solid formulation of Term 6 wherein the water-soluble additive agent is sodium chloride, glucose, mannitol, lactose, glycine, sodium cholate, sodium desoxycholate and/or sodium glycocholate.
Term 8. The parenteral solid formulation of Term 6 wherein the water-soluble additive agent is sodium chloride and/or sodium desoxycholate.

Term 9. The parenteral solid formulation of Term 8 wherein the hardly water-soluble substance is low substituted hydroxypropylcellulose, and the water-soluble additive agent is sodium chloride.

Term 10. The parenteral solid formulation of Term 8 wherein the hardly water-soluble substance is cholesterol, and the water-soluble additive agent is sodium chloride and sodium desoxycholate.

Term 11. The parenteral solid formulation of any one of Terms 1 to 10 wherein the total weight of the water-soluble drug, the hardly water-soluble substance and the optional water-soluble additive agent is 10 to 40 % per the whole weight of the formulation, provided that the total weight of the water-soluble drug and the water-soluble additive agent is not more than 35 % per the whole weight of the formulation.

Term 12. The parenteral solid formulation of any one of Terms 1 to 11 comprising essentially a water-soluble drug, a pharmaceutically acceptable hardly water-soluble solid substance, and an optional water-soluble additive agent, and which comprises silicone as a carrier.

Term 13. The parenteral solid formulation of any one of Terms 1 to 12 which is a matrix formulation.

Term 14. The parenteral solid formulation of any one of Terms 1 to 13 which is an implantable formulation.
Term 15. The parenteral solid formulation of any one of Terms 1 to 14 wherein the water-soluble drug does not include a compound of formula (1): 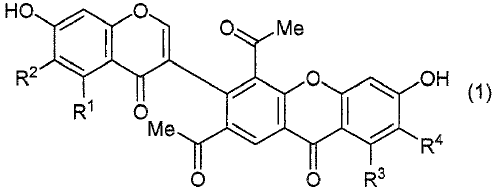 wherein R¹ is hydrogen atom or carboxyl group, R² is hydrogen atom or hydroxyl group, R³ is hydrogen atom or carboxyl group, and R⁴ is hydrogen atom or hydroxyl group, or a pharmaceutically acceptable salt thereof.

### BRIEF DESCRIPTION OF THE DRAWINGS

- [Figure 1]: Results of Test Example 1 are shown.
- [Figure 2]: Results of Test Example 2 are shown.
- [Figure 3]: Results of Test Example 3 are shown.
- [Figure 4]: Results of Test Example 4 are shown.
- [Figure 5]: Results of Test Example 5 are shown.
- [Figure 6]: Results of Test Example 6 are shown.
- [Figure 7]: Results of Test Example 7 are shown.
- [Figure 8]: Results of Test Example 8 are shown.
- [Figure 9]: Results of Test Example 9 are shown.
- [Figure 10]: Results of Test Example 10 are shown.
- [Figure 11]: Results of Test Example 11 are shown.
- [Figure 12]: Results of Test Example 12 are shown.
- [Figure 13]: Results of Test Example 13 are shown.
- [Figure 14]: Results of Test Example 14 are shown.
- [Figure 15]: Results of Test Example 15 are shown.
- [Figure 16]: Results of Test Example 16 are shown.
- [Figure 17]: Results of Test Example 17 are shown.
- [Figure 18]: Results of Test Example 18 are shown.
- [Figure 19]: Results of Test Example 19 are shown.
- [Figure 20]: Results of Test Example 20 are shown.
- [Figure 21]: Results of Test Example 21 are shown.

### DESCRIPTION OF EMBODIMENTS

The water-soluble drug used herein means a drug which has a low solubility in silicone and cannot be expected to substantially release the drug in silicone by diffusion, and intends that the aqueous solubility thereof is 0.1 mg/mL or more and n-octanol/water partition coefficient thereof is less than 10 (*i.e*. Log P as a common logarithm of n-octanol/water partition coefficient is less than 1) as a rough guide.
Acetaminophen, zonisamide, metformin, L-phenylalanine, tranexamic acid, and droxidopa used in the following Examples have Log P of 0.5, 0.2, -1.3, -1.5, -2.0, and, - 3.2, respectively. Furthermore, the water-soluble drug which can be applied to the present invention is not limited on the basis of the molecular weight thereof, and includes compounds having, for example, high molecular weight such as albumin (molecular weight: about 66000) and low molecular weight such as acetaminophen (molecular weight: 151). In addition, in case that the water-soluble drug used herein is a low molecular weight, Log P is preferably -3 or more but less than 1. On the other hand, in case that the water-soluble drug used herein is a high molecular weight, the preferred range of Log P is not specifically limited.
The water-soluble drug used herein is not specifically limited, but includes preferably a water-soluble drug which is desirable for a long-term sustained administration, more preferably a water-soluble drug which further has high activity in a small amount. The water-soluble drug used herein includes, for example, water-soluble drugs used as an antitumor drug, antibiotic, anti-inflammatory drug, immunosuppressive agent, medicament for nervous system, tissue-remodeling accelerator, and cytoprotectant. In more specific, the water-soluble drug used herein includes, for example, cytokines such as interferon and interleukin; hematopoietic growth factors such as colony-stimulating factors and erythropoietin; hormones such as growth hormones, growth hormone-releasing factors, calcitonin, luteinizing hormones, and luteinizing hormone-releasing hormones; growth factors such as somatomedin, nerve growth factors, neurotrophic factors, fibroblast growth factors, and hepatocyte growth factors; bone-metabolism-related proteins such as BMP (Bone Morphogenetic Protein); cell-adhesion factors; immunosuppressive agents; enzymes such as asparaginase, superoxide dismutase, tissue plasminogen activator, urokinase, and pro-urokinase; and antigens, adjuvants, cancer antigens, nucleic acid, adriamycin, bleomycin, mitomycin, fluorouracil, peplomycin sulfate, daunorubicin hydrochloride, hydroxyurea, neocarzinostatin, sizofiran, estramustine phosphate sodium, carboplatin, β-lactams, tetracyclines, aminoglycosides, fosfomycin, acetaminophen, levodopa, droxidopa, metronidazole, antipyrine, metformin, zonisamide, haloperidol, tranexamic acid, L-phenylalanine and the like which may be used as an inhibitor, antibody, vaccine for humans and/or animals of the above-mentioned cytokines, growth factors, and enzymes. In additon, the present formulation may comprise several drugs depending on the disease and applying method.
The "pharmaceutically acceptable salt" in Term 15 represents a medically or veterinary acceptable salt and includes, for example, inorganic basic salts such as sodium salts, potassium salts, calcium salts, magnesium salts, aluminum salts, and ammonium salts; organic basic salts such as triethylammonium salts, triethanolammonium salts, pyridinium salts, and diisopropylammonium salts; and basic amino acid salts such as arginine and lysine salts. For example, in case that the compound of Formula (1) has two carboxyl groups, the pharmaceutically acceptable salt includes, for example, monosodium/monopotassium salt.

The hardly water-soluble solid substance used herein is not specifically limited and includes a medically/pharmaceutically acceptable substance which is solid at room temperature and slightly soluble in an in vivo environment (*i.e.* at a neutral pH and 37°C). In specific, the hardly water-soluble solid substance includes a substance which requires 100 mL or more, preferably 1000 mL or more, of water to dissolve 1 g of thereof. The hardly water-soluble solid substance includes, for example, swellable polymers used as a disintegrant for an oral preparation; and such swellable polymers include, for example, low substituted hydroxypropylcellulose (L-HPC) which contains 5 to 16 % of hydroxypropoxyl group, partly pregelatinized starch, crospovidone, croscarmellose sodium (crosCMC-Na), carmellose calcium, sodium carboxymethyl starch, hydroxypropyl starch; preferably L-HPC, partly pregelatinized starch, crospovidone, crosCMC-Na; and more preferably L-HPC, partly pregelatinized starch, crosCMC-Na. Other than swellable polymers, the hardly water-soluble substance includes, for example, fatty acids which are solid at room temperature (*e.g*. myristic acid, lauric acid and palmitic acid), cholesterol, and saccharin; and preferably myristic acid, cholesterol, and saccharin. One or several different types of the above-listed hardly water-soluble substances may be contained in the solid formulation of the present invention. The especially preferred hardly water-soluble substance is L-HPC and/or cholesterol. The cholesterol can be used together with a substance which helps the dissolution thereof (*e.g*. bile salt) to achieve further preferred effects.

The present invention may comprise a water-soluble additive agent in order to, for example, optimize the release rate or stabilize the drug. The water-soluble additive agent used herein is solid at room temperature, and 1 g thereof is dissolved in water of less than 100 mL, preferably less than 10 mL in an *in vivo* environment (*i.e.* at a neutral pH and 37°C). The water-soluble additive agent used herein is not limited as long as it is medically/pharmaceutically acceptable and includes, for example, saccharides, salts, amino acids, and bile salts. In specific, the saccharides used herein include, for example, glucose, mannitol, lactose, trehalose, sucrose, erythritol, sorbitol and xylitol; and preferably glucose, mannitol and lactose. The salts used herein include, for example, sodium chloride, potassium chloride and calcium chloride; and preferably sodium chloride. The amino acids used herein include 20 different α-amino acids which occur in nature such as glycine, alanine, proline, serine, arginine and glutamic acid; and preferably glycine. The bile salts used herein include, for example, primary bile salts such as sodium cholate and sodium chenodeoxy cholate, secondary bile salts such as sodium desoxycholate and sodium lithocholate, and conjugated bile salts such as sodium glycocholate and sodium taurocholate; and preferably sodium cholate, sodium desoxycholate and sodium glycocholate. More preferably, the water-soluble additive agent is sodium chloride and/or sodium desoxycholate. One or several different types of the above-listed water-soluble additive agents may be contained in the solid formulation of the present invention.
In case that cholesterol is used as the hardly water-soluble substance, it is preferable to combine a substance which helps the dissolution thereof, as described above. In specific, cholesterol can be combined with preferably the above-mentioned bile salts; more preferably sodium cholate, sodium desoxycholate or sodium glycocholate; and the most preferably sodium desoxycholate.

Silicone shows an excellent biocompatibility and has been successfully used as a material for artificial organs and medical devices for a long time. Silicone can exist in various states such as oil, gel and rubber, depending on the polymerization degree of siloxane bonds and substituents induced in the silicone. The silicone used herein is not specifically limited as long as it is a solid, and such a solid may be made by curing oil-state or gel-state silicone. The silicone used herein may be, for example, SILASTIC Q7-4750 of polydimethylsiloxane (manufactured by Dow Corning Corp.) and MED-4750 (manufactured by Nusil Corp.).

The "(formulation) comprising essentially a water-soluble drug, a pharmaceutically acceptable hardly water-soluble solid substance, and an optional water-soluble additive agent, and which comprises silicone as a carrier" used herein means that the formulation comprises the above-mentioned components as main components, and may also comprise in a small amount some additional components as long as they do not have an adverse impact on the effects of the present formulation. The total weight of "the water-soluble drug, pharmaceutically acceptable hardly water-soluble solid substance, optional water-soluble additive agent, and silicone as a carrier" is 95 % or more, for example, 96 % or more, 97 % or more, 98 % or more, 99 % or more, and 100 % by weight per the whole weight of the formulation. Other than the above-mentioned components, there are basically no components essential for the present formulation, but the formulation may optionally comprise, for example, agents to adjust the curing rate of silicone when manufacturing, substances to adjust the intensity or flexibility of the formulation, and radiopaque markers to identify the place of the implanted formulation by X-ray examination. The radiopaque marker used herein includes, for example, platinum alloys such as platinum, platinum/iridium and platinum/nickel, and palladium alloys.

The shape and size of the parenteral solid formulation used herein is not limited as long as it is suitable for the placement in the treatment site. The shape of the formulation used herein includes, for example, rod-, needle-, string-, coil-, cone-, screw-, button-, sphere-, hemisphere-, and sheet-like shapes; preferably rod- and sheet-like shapes. Regarding the size, the formulation used herein has a diameter or thickness of approximately 0.1 mm to 10 cm and typically 0.3 mm to 3 cm as a maximum length thereof.
The parenteral solid formulation used herein includes transdermal formulations (e.g. tape formulations) and implantable formulations, and preferably implantable formulations.
In addition, the matrix formulation used herein is a controlled-release formulation wherein the powder pharmaceuticals and additives are homogeneously dispersed in the silicone which is a carrier.

Without reference to a specific theory, it is perceived that a formulation comprising a hardly water-soluble solid substance can attain a controlled release of the water-soluble drug for a long period for the following reason.
In releasing a water-soluble drug from silicone, firstly the formulation is continuously dissolved from the surface thereof to form a channel comprising water, and then the drug is dissolved and diffused in the channel with releasing the drug. In channel forming, the conventionally used water-soluble additive agents (hydrophilic ingredients) are rapidly dissolved and voids corresponding to the particle volume of the additive agent are formed inside the formulation, and thus a large channel is suddenly formed in a continuous manner. Accordingly, the release is remarkably accelerated and the formulation shows an initial burst with a short-term release. On the other hand, in case of the present formulation comprising a hardly water-soluble solid substance, a large void is not suddenly formed because the rate of dissolution is slow. Thus, the region of the dissolved additive-agent serves as a pathway to deliver a suitable amount of water and water-soluble drug, and it is therefore possible to attain a controlled release for a long period.

The water-soluble drug is contained in an amount of 42 % or less, preferably 35 % or less, more preferably 20 % or less, and the most preferably 15 % or less by weight per the whole weight of the formulation.
The silicone is contained in an amount of 55 % or more, preferably 60 to 90 %, and more preferably 65 to 85 % by weight per the whole weight of the formulation.
The hardly water-soluble solid substance is contained in an amount of 3 to 35 %, preferably 3 to 28 % by weight per the whole weight of the formulation. Among the hardly water-soluble substance, a preferred amount of the low substituted hydroxypropylcellulose is 3 to 28 %, and that of the cholesterol is 4 to 20 %. A formulation having the above-mentioned contents can accomplish a small initial-burst, and a sustained-release of the water-soluble drug for a long period of 2 weeks or more.
The optional water-soluble additive agent is contained in an amount of less than 35 %, preferably 20 % or less and more preferably 16 % or less by weight per the whole weight of the formulation.

The content of the water-soluble drug, hardly water-soluble solid substance and optional water-soluble additive agent is determined in relation to the content of silicone. If the total content of the components other than silicone is too low, the channel forming which serves as a release mechanism may not be enough, and thus the drug may not be sufficiently released out from the formulation. Meanwhile, if the total content of the components other than silicone is too high, the silicone formulation may cause trouble in shaping or decrease in intensity. Thus, the content of silicone is adjusted to 55 % or more, that is, the total content of the components other than silicone is adjusted to 45 % or less, preferably 10 to 40 % by weight per the whole weight of the formulation.
Furthermore, from the viewpoint of controlling the release, if the total content of water-soluble materials is too high, the release will be too fast and end up in a burst release. Thus, it is desirable that the total content of the water-soluble drug and water-soluble additive agent as the water-soluble material is not more than 35 %.

The particle size of the water-soluble drug, hardly water-soluble solid substance and optional water-soluble additive agent, which are dispersed in silicone as a powder, may have some effects on the release profile. Thus, in order to keep the same quality, it is desirable to optionally control the particle size within a certain range; and typically, the upper limit of the particle size is controlled to 300 µm or less, more preferably 200 µm or less.

### (Effect of the Invention)

The present formulation having a compact sheet- or rod-like shape can be used as an implantable formulation.

### EXAMPLES

Hereinafter, the present invention is explained in more detail by illustrating Examples and Comparative Examples, and Test Examples thereof. BSA (bovine serum albumin) and HSA (human serum albumin) are used herein as an example of the water-soluble high-molecular-weight drug; and acetaminophen, zonisamide, metformin hydrochloride, L-phenylalanine, tranexamic acid, and droxidopa are used herein as an example of the water-soluble low-molecular-weight drug. However, the scope of the present invention should be limited to neither the above-mentioned drugs nor the materials/devices used herein.

### (Source)

L-HPC (Shin-Etsu Chemical Co., Ltd.)
Partly pregelatinized starch (Asahi Kasei Corp.) Crospovidone (BASF Japan Ltd.)
Cros CMC-Na (FMC BioPolymer)
CMC-Na (Nacalai Tesque, Inc.)
BSA (Nacalai Tesque, Inc.)
HSA (Nacalai Tesque, Inc.)
Crystalline sodium chloride (Nacalai Tesque, Inc.)
Glycine (Nacalai Tesque, Inc.)
Glucose (Nacalai Tesque, Inc.)
Mannitol (Towa-Kasei Co., Ltd.)
Lactose (DMV)
Cholesterol (Kanto Chemical Co., Inc.)
Acetaminophen (Nacalai Tesque, Inc.)
Zonisamide (*e.g*. prepared according to the process disclosed in US 4,172,896 B)
Metformin hydrochloride (*e.g*. prepared according to the process disclosed in FR 2322860 B)
L-phenylalanine (Nacalai Tesque, Inc.)
Tranexamic acid (Nacalai Tesque, Inc.)
Droxidopa [*e.g*. prepared according to the process disclosed in JP 5(1993)-020425 A]
PEG4000 (Nacalai Tesque, Inc.)

### Example 1

L-HPC (60 mg) and BSA (bovine serum albumin) (60 mg) were mixed in a mortar to give a mixed powder. SILASTIC Q7-4750 Silicone A component (140 mg) and Silicone B component (140 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.3 mm. The sheet formulation was cut into a size of 5 mm x 7 mm to give Formulation 1.

### Example 2

Crystalline sodium chloride was milled in a mortar to adjust the particle diameter thereof to 100 µm or less, which was measured with a light microscope (phase-contrast microscope BX-51-33-PHU-D, OLYMPAS). To the above-obtained sodium chloride (12 mg) were added L-HPC (48 mg) and then BSA (60 mg), and the three kinds of powder were mixed all together in the mortar to give a mixed powder. SILASTIC Q7-4750 Silicone A component (140 mg) and Silicone B component (140 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.3 mm. The sheet formulation was cut into a size of 5 mm x 7 mm to give Formulation 2.

### Test Example 1

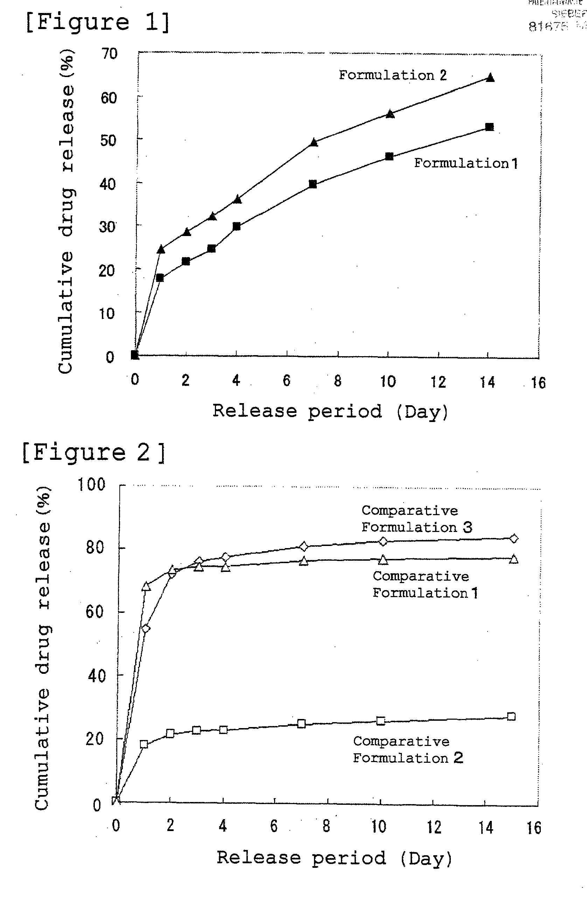

Each of the formulations obtained in Examples 1 and 2 (see, Table 1) was put into phosphate buffered saline (PBS) (1 mL), kept at 37°C, measured the BSA released from the formulation with an ultraviolet spectrophotometer (SPECTROPHOTOMETER DU 800, BECKMAN COULTER, detection wavelength 280 nm, at room temperature), and the value was calculated to determine the cumulative percentage of drug release. The results are shown in Figure 1. The present formulations attained an excellent sustained-release lasting for 2 weeks.

**[Table 1]**

| | Dosage form | Drug | Concentration of Drug (wt%) | Concentration of L-HPC (wt%) | Other additive agents |
|---|---|---|---|---|---|
| Formulation 1 | Sheet | BSA | 15 | 15 | None |
| Formulation 2 | Sheet | BSA | 15 | 12 | NaCl 3 wt% |

### Comparative Example 1

SILASTIC Q7-4750 Silicone A component (140 mg) and Silicone B component (140 mg) manufactured by Dow Corning were kneaded together with a twin roll. After the above silicones were kneaded, BSA (120 mg) was quickly added thereto, and the mixture was kneaded. Then, the kneaded product was rolled into a sheet shape with a twin roll and cured at 40°C for 1 day to give a sheet formulation with a thickness of 0.4 mm. The sheet formulation was cut into a size of 5 mm x 7 mm to give Comparative Formulation 1.

### Comparative Example 2

SILASTIC Q7-4750 Silicone A component (170 mg) and Silicone B component (170 mg) manufactured by Dow Corning were kneaded together with a twin roll. After the above silicones were kneaded, BSA (60 mg) was quickly added thereto, and the mixture was kneaded. Then, the kneaded product was rolled into a sheet shape with a twin roll and cured at 40°C for 1 day to give a sheet formulation with a thickness of 0.4 mm. The sheet formulation was cut into a size of 5 mm x 7 mm to give Comparative Formulation 2.

### Comparative Example 3

CMC-Na (60 mg) and BSA (60 mg) were mixed in a mortar to give a mixed powder. SILASTIC Q7-4750 Silicone A component (140 mg) and Silicone B component (140 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.4 mm. The sheet formulation was cut into a size of 5 mm x 7 mm to give Comparative Formulation 3.

### Test Example 2

Each of the formulations obtained in Comparative Examples 1 to 3 (see, Table 2) was tested in the same manner as Test Example 1 to determine the cumulative percentage of drug release of BSA. The results are shown in Figure 2.
Comparative Formulation 1, which contains BSA in an amount of 30 wt% without any additive agents, showed a marked initial burst and released most of the drug on Day 1, and thus no sustained release was observed after that. Comparative Formulation 2, which contains BSA in an amount of 15 wt% without any additive agents like Comparative Formulation 1, showed a small initial burst but no sustained release was observed, and thus the cumulative percentage of drug release remained low. Comparative Formulation 3, which does not contain the hardly water-soluble solid substance of the present invention but instead contains 15 % of CMC-Na disclosed as an additive agent in Patent Reference 4, showed a rapid release without any sustained release, like Comparative Formulation 1.

**[Table 2]**

| | Dosage form | Drug | Concentration of Drug (wt%) | Other additive agents | Notes |
|---|---|---|---|---|---|
| Comparative Formulation 1 | Sheet | BSA | 30 | None | Initial burst, no sustained-release |
| Comparative Formulation 2 | Sheet | BSA | 15 | None | Decrease of release |
| Comparative Formulation 3 | Sheet | BSA | 15 | CMC-Na 15 wt% | The additive agent is disclosed in Patent Document 4 |

### Example 3

Cholesterol (120 mg) and HSA (60 mg) were mixed in a mortar to give a mixed powder. SILASTIC Q7-4750 Silicone A component (210 mg) and Silicone B component (210 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.8 mm. The sheet formulation was cut into a size of 5 mm x 7 mm to give Formulation 3.

### Test Example 3

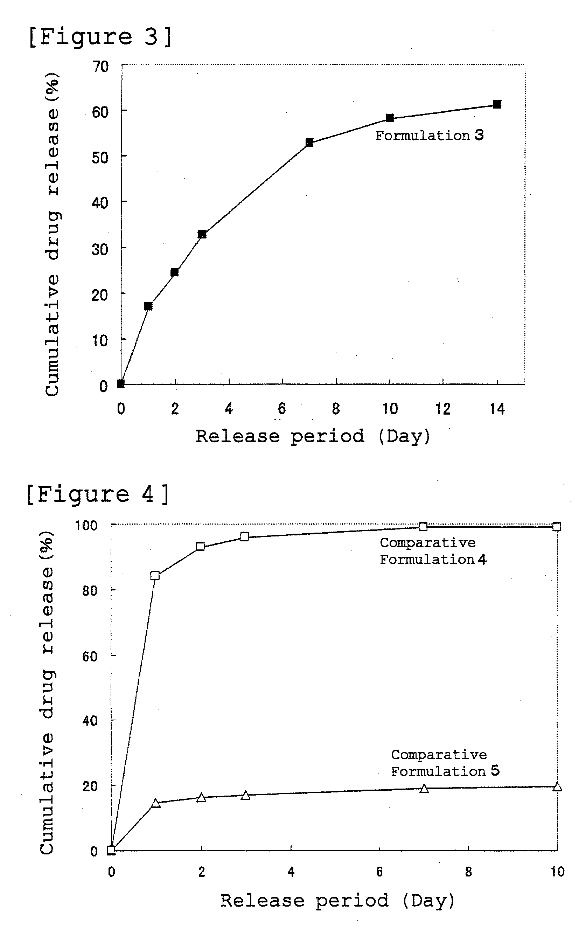

Formulation 3 obtained in Example 3 (see, Table 3) was put into PBS (1 mL), kept at 37°C, measured the HSA released from the formulation with an ultraviolet spectrophotometer, and the value was calculated to determine the cumulative percentage of drug release. As shown in Figure 3, the formulation attained an excellent sustained-release lasting for 2 weeks.

**[Table 3]**

| | Dosage form | Drug | Concentration of Drug (wt%) | Cholesterol (wt%) | Other additive agents |
|---|---|---|---|---|---|
| Formulation 3 | Sheet | HSA | 10 | 20 | None |

### 1 Comparative Example 4

SILASTIC Q7-4750 Silicone A component (210 mg) and Silicone B component (210 mg) manufactured by Dow Corning were kneaded together with a twin roll. After the above silicones were kneaded, HSA (180 mg) was quickly added thereto, and the mixture was kneaded. Then, the kneaded product was rolled into a sheet shape with a twin roll and cured at 40°C for 1 day to give a sheet formulation with a thickness of 0.8 mm. The sheet formulation was cut into a size of 5 mm x 7 mm to give Comparative Formulation 4.

### Comparative Example 5

SILASTIC Q7-4750 Silicone A component (270 mg) and Silicone B component (270 mg) manufactured by Dow Corning were kneaded together with a twin roll. After the above silicones were kneaded, HSA (60 mg) was quickly added thereto, and the mixture was kneaded. Then, the kneaded product was rolled into a sheet shape with a twin roll and cured at 40°C for 1 day to give a sheet formulation with a thickness of 0.8 mm. The sheet formulation was cut into a size of 5 mm x 7 mm to give Comparative Formulation 5.

### Test Example 4

Each of the formulations obtained in Comparative Examples 4 and 5 (see, Table 4) was tested in the same manner as Test Example 3 to determine the cumulative percentage of drug release of HSA. The results are shown in Figure 4.
Comparative Formulation 4, which contains HSA in an amount of 30 % without any additive agents, showed a marked initial burst and released most of the drug by Day 2, and thus no sustained release was observed after that. Comparative Formulation 5, which contains HSA in an amount of 10 % without any additive agents like Comparative Formulation 4, showed a smaller initial burst but no sustained release was observed, and thus the cumulative percentage of drug release remained low.

**[Table 4]**

| | Dosage form | Drug | Concentration of Drug (wt%) | Additive agents | Results |
|---|---|---|---|---|---|
| Comparative Formulation 4 | Sheet | HSA | 30 | None | Initial burst, no sustained-release |
| Comparative Formulation 5 | Sheet | HSA | 10 | None | Decrease of release |

### Example 4

L-HPC (80 mg) and acetaminophen (40 mg) were mixed in a mortar to give a mixed powder. SILASTIC Q7-4750 Silicone A component (140 mg) and Silicone B component (140 mg) manufactured by Dow Corning were kneaded together with a twin roll. After the above silicones were kneaded, all of the above-obtained mixed powder was quickly added thereto, and the mixture was kneaded. The kneaded product was extruded through a plastic syringe manufactured by Terumo Corporation, and then cured at 40°C for 1 day to give a rod formulation with a diameter of 2.0 mm. The rod formulation was cut into a length of 7 mm to give Formulation 4.

### Example 5

L-HPC (112 mg) and acetaminophen (8 mg) were mixed in a mortar to give a mixed powder. SILASTIC Q7-4750 Silicone A component (140 mg) and Silicone B component (140 mg) manufactured by Dow Corning were kneaded together with a twin roll. After the above silicones were kneaded, all of the above-obtained mixed powder was quickly added thereto, and the mixture was kneaded. The kneaded product was extruded through a nozzle, and then cured at 40°C for 1 day to give a rod formulation with a diameter of 1.9 mm. The rod formulation was cut into a length of 7 mm to give Formulation 5.

### Example 6

To lactose (8 mg) were added L-HPC (104 mg) and then acetaminophen (8 mg) in a mortar, and the three kinds of powder were mixed all together to give a mixed powder. SILASTIC Q7-4750 Silicone A component (140 mg) and Silicone B component (140 mg) manufactured by Dow Corning were kneaded together with a twin roll. After the above silicones were kneaded, all of the above-obtained mixed powder was quickly added thereto, and the mixture was kneaded. The kneaded product was extruded through a nozzle, and then cured at 40°C for 1 day to give a rod formulation with a diameter of 1.9 mm. The rod formulation was cut into a length of 7 mm to give Formulation 6.

### Test Example 5

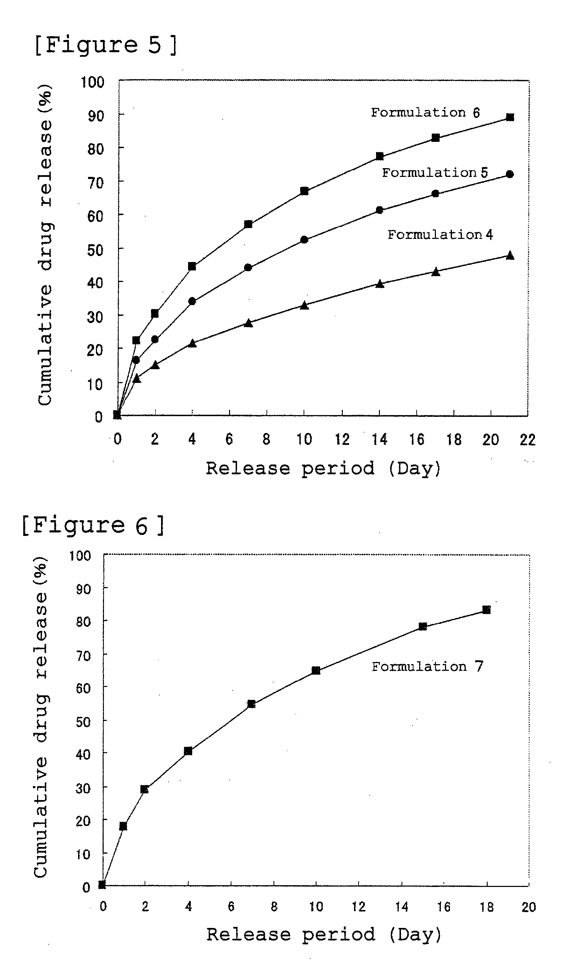

Each of the formulations obtained in Examples 4 to 6 (see, Table 5) was put into PBS (1 mL), kept at 37°C, measured the acetaminophen (AA) released from the formulation with an ultraviolet spectrophotometer, and the value was calculated to determine the cumulative percentage of drug release. The results are shown in Figure 5.
Formulations 4 and 5 contain acetaminophen in an amount of 10 % and 2 % respectively, and both of the formulations attained an excellent sustained-release lasting for 3 weeks. Formulation 6, which further contains lactose as a water-soluble additive agent in an amount of 2 %, attained a sustained-release and also exhibited an enhanced-release derived from combining the water-soluble additive agent.

**[Table 5]**

| | Dosage form | Drug | Concentration of Drug (wt%) | Concentration of L-HPC (wt%) | Other additive agents |
|---|---|---|---|---|---|
| Formulation 4 | Rod | AA | 10 | 20 | None |
| Formulation 5 | Rod | AA | 2 | 28 | None |
| Formulation 6 | Rod | AA | 2 | 26 | Lactose 2 wt% |

### Example 7

To glycine (8 mg) were added L-HPC (104 mg) and then acetaminophen (8 mg) in a mortar, and the three kinds of powder were mixed all together to give a mixed powder. SILASTIC Q7-4750 Silicone A component (140 mg) and Silicone B component (140 mg) manufactured by Dow Corning were kneaded together with a twin roll. After the above silicones were kneaded, all of the above-obtained mixed powder was quickly added thereto, and the mixture was kneaded. The kneaded product was extruded through a nozzle, and then cured at 40°C for 1 day to give a rod formulation with a diameter of 2.0 mm. The rod formulation was cut into a length of 7 mm to give Formulation 7.

### Test Example 6

Formulation 7 obtained in Example 7 (see, Table 6) was tested in the same manner as Test Example 5 to determine the cumulative percentage of drug release of acetaminophen (AA). As shown in Figure 6, the formulation attained an excellent sustained-release lasting for 18 days.

**[Table 6]**

| | Dosage form | Drug | Concentration of Drug (wt%) | Concentration of L-HPC (wt%) | Other additive agents |
|---|---|---|---|---|---|
| Formulation 7 | Rod | AA | 2 | 26 | Glycine (2 wt%) |

### Example 8

Crystalline sodium chloride was milled in a mortar to adjust the particle diameter thereof to 100 µm or less. To the above-obtained sodium chloride (18 mg) were added L-HPC (102 mg) and then acetaminophen (60 mg), and the three kinds of powder were mixed all together in the mortar to give a mixed powder. SILASTIC Q7-4750 Silicone A component (210 mg) and Silicone B component (210 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.8 mm. The sheet formulation was cut into a size of 5 mm x 7 mm to give Formulation 8.

### Test Example 7

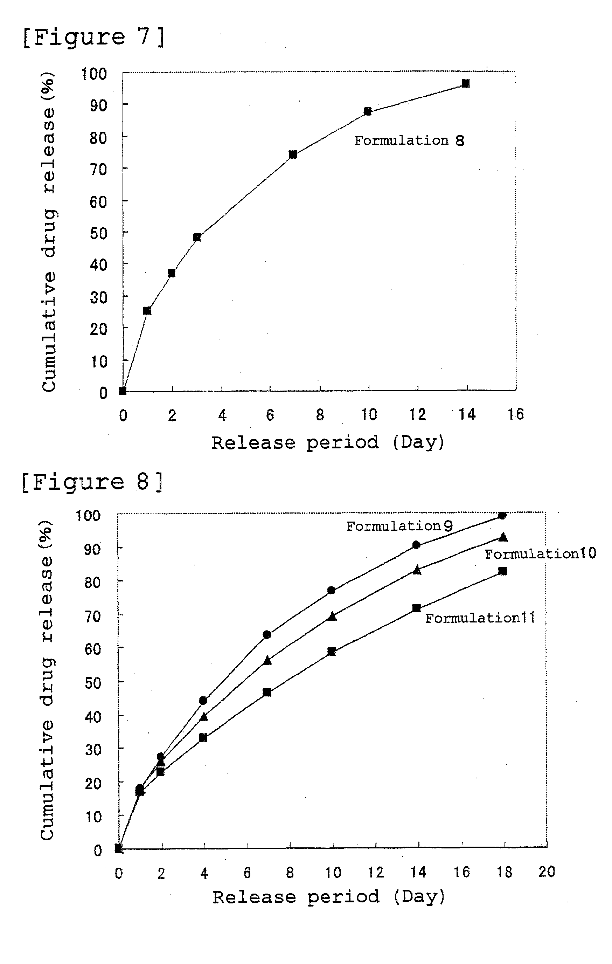

Formulation 8 obtained in Example 8 (see, Table 7) was kept at 37°C in PBS (1 mL), measured the acetaminophen (AA) released from the formulation with an ultraviolet spectrophotometer, and the value was calculated to determine the cumulative percentage of drug release. As shown in Figure 7, the formulation attained an excellent sustained-release lasting for 2 weeks.

**[Table 7]**

| | Dosage form | Drug | Concentration of Drug (wt%) | Concentration of L-HPC (wt%) | Other additive agents |
|---|---|---|---|---|---|
| Formulation 8 | Sheet | AA | 10 | 17 | NaCl (3 wt%) |

### Example 9

Crystalline sodium chloride was milled in a mortar to adjust the particle diameter thereof to 100 µm or less. To the above-obtained sodium chloride (30 mg) were added L-HPC (100 mg) and then acetaminophen (100 mg), and the three kinds of powder were mixed all together in the mortar to give a mixed powder. SILASTIC Q7-4750 Silicone A component (385 mg) and Silicone B component (385 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.8 mm. The sheet formulation was cut into a size of 5 mm x 7 mm to give Formulation 9.

### Example 10

Crystalline sodium chloride was milled in a mortar to adjust the particle diameter thereof to 100 µm or less. To the above-obtained sodium chloride (30 mg) were added L-HPC (50 mg) and then acetaminophen (100 mg), and the three kinds of powder were mixed all together in the mortar to give a mixed powder. SILASTIC Q7-4750 Silicone A component (410 mg) and Silicone B component (410 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.8 mm. The sheet formulation was cut into a size of 5 mm x 7 mm to give Formulation 10.

### Example 11

Crystalline sodium chloride was milled in a mortar to adjust the particle diameter thereof to 100 µm or less. To the above-obtained sodium chloride (30 mg) were added L-HPC (30 mg) and then acetaminophen (100 mg), and the three kinds of powder were mixed all together in the mortar to give a mixed powder. SILASTIC Q7-4750 Silicone A component (420 mg) and Silicone B component (420 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.8 mm. The sheet formulation was cut into a size of 5 mm x 7 mm to give Formulation 11.

### Test Example 8

Each of the formulations obtained in Examples 9 to 11 (see, Table 8) was tested in the same manner as Test Example 7 to determine the cumulative percentage of drug release of acetaminophen (AA). The results are shown in Figure 8. Each of the formulations contains L-HPC in a different amount and also NaCl as a water-soluble additive agent in an amount of 3 wt%, but all of the formulations attained an excellent sustained-release lasting for 18 days.

**[Table 8]**

| | Dosage form | Drug | Concentration of Drug (wt%) | Concentration of L-HPC (wt%) | Other additive agents |
|---|---|---|---|---|---|
| Formulation 9 | Sheet | AA | 10 | 10 | NaCl (3 wt%) |
| Formulation 10 | Sheet | AA | 10 | 5 | NaCl (3 wt%) |
| Formulation 11 | Sheet | AA | 10 | 3 | NaCl (3 wt%) |

### Example 12

Crystalline sodium chloride was milled in a mortar to adjust the particle diameter thereof to 100 µm or less. To the above-obtained sodium chloride (12 mg) were added sodium desoxycholate (34 mg), cholesterol (34 mg) and then acetaminophen (40 mg), and the four kinds of powder were mixed all together in the mortar to give a mixed powder. SILASTIC Q7-4750 Silicone A component (140 mg) and Silicone B component (140 mg) manufactured by Dow Corning were kneaded together with a twin roll. After the above silicones were kneaded, all of the above-obtained mixed powder was quickly added thereto, and the mixture was kneaded. The kneaded product was extruded through a nozzle, and then cured at 40°C for 1 day to give a rod formulation with a diameter of 2.1 mm. The rod formulation was cut into a length of 7 mm to give Formulation 12.

### Example 13

Crystalline sodium chloride was milled in a mortar to adjust the particle diameter thereof to 100 µm or less. To the above-obtained sodium chloride (12 mg) were added sodium desoxycholate (52 mg), cholesterol (16 mg) and then acetaminophen (40 mg), and the four kinds of powder were mixed all together in the mortar to give a mixed powder. SILASTIC Q7-4750 Silicone A component (140 mg) and Silicone B component (140 mg) manufactured by Dow Corning were kneaded together with a twin roll. After the above silicones were kneaded, all of the above-obtained mixed powder was quickly added thereto, and the mixture was kneaded. The kneaded product was extruded through a nozzle, and then cured at 40°C for 1 day to give a rod formulation with a diameter of 1.9 mm. The rod formulation was cut into a length of 7 mm to give Formulation 13.

### Test Example 9

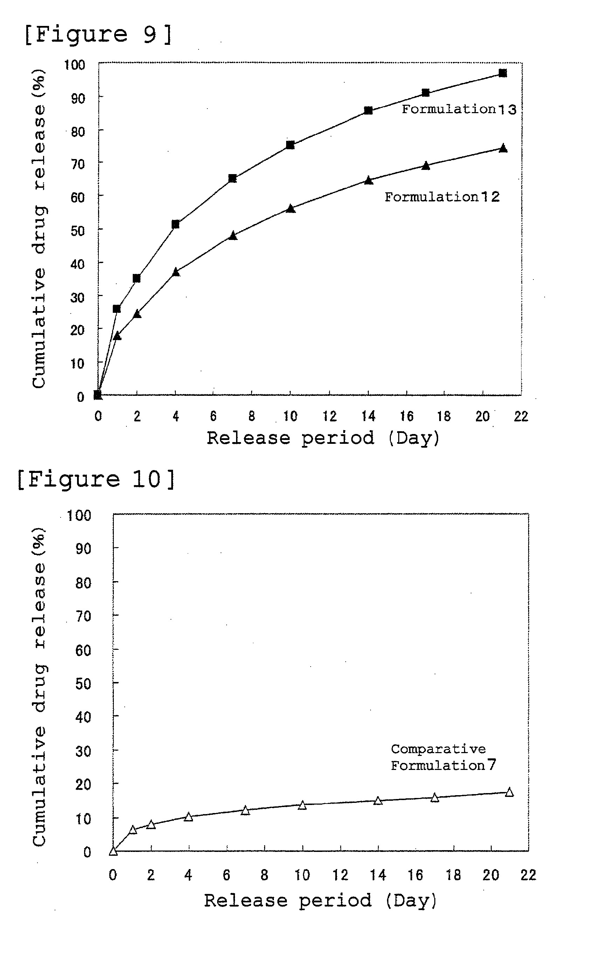

Each of the formulations obtained in Examples 12 and 13 (see, Table 9) was tested in the same manner as Test Example 5 to determine the cumulative percentage of drug release of acetaminophen (AA). The results are shown in Figure 9. Each of the formulations contains cholesterol as the hardly water-soluble solid substance, and sodium desoxycholate and NaCl as the water-soluble additive agent; and both of the formulations attained an excellent sustained-release lasting for 3 weeks.

**[Table9]**

| | Dosage form | Drug | Concentration of drug (wt%) | Concentration of cholesterol (wt%) | Other additive agents |
|---|---|---|---|---|---|
| Formulation 12 | Rod | AA | 10 | 8.5 | Sodium desoxycholate 8.5 wt% + NaCl 3 wt% |
| Formulation 13 | Rod | AA | 10 | 4 | Sodium desoxycholate 13 wt% + NaCl 3 wt% |

### Comparative Example 7

SILASTIC Q7-4750 Silicone A component (140 mg) and Silicone B component (140 mg) manufactured by Dow Corning were kneaded together with a twin roll. After the above silicones were kneaded, acetaminophen (120 mg) was quickly added thereto, and the mixture was kneaded. The kneaded product was extruded through a nozzle, and then cured at 40°C for 1 day to give a rod formulation with a diameter of 2.0 mm. The rod formulation was cut into a length of 7 mm to give Comparative Formulation 7.

### Test Example 10

Comparative Formulation 7 obtained in Comparative Example 7 (see, Table 10) was tested in the same manner as Test Example 5 to determine the cumulative percentage of drug release of acetaminophen (AA). The results are shown in Figure 10. The comparative formulation, which does not contain any additive agents, showed a slow release-rate and the cumulative percentage of drug release of acetaminophen remained low.

**[Table 10]**

| | Dosage form | Drug | Concentration of Drug (wt%) | Additive agents | Result or note |
|---|---|---|---|---|---|
| Comparative Formulation 7 | Rod | AA | 30 | None | Decrease of release |

### Comparative Example 8

Crystalline sodium chloride was milled in a mortar to adjust the particle diameter thereof to 100 µm or less. To the above-obtained sodium chloride (112 mg) was added acetaminophen (8 mg), and the two kinds of powder were mixed all together in the mortar to give a mixed powder. SILASTIC Q7-4750 Silicone A component (140 mg) and Silicone B component (140 mg) manufactured by Dow Corning were kneaded together with a twin roll. After the above silicones were kneaded, all of the above-obtained mixed powder was quickly added thereto, and the mixture was kneaded. The kneaded product was extruded through a nozzle, and then cured at 40°C for 1 day to give a rod formulation with a diameter of 2.0 mm. The rod formulation was cut into a length of 7 mm to give Comparative Formulation 8.

### Comparative Example 9

A water-soluble HPC (NISSO, SL NCl-0611) (112 mg) and acetaminophen (8 mg) were mixed in a mortar to give a mixed powder. SILASTIC Q7-4750 Silicone A component (140 mg) and Silicone B component (140 mg) manufactured by Dow Corning were kneaded together with a twin roll. After the above silicones were kneaded, all of the above-obtained mixed powder was quickly added thereto, and the mixture was kneaded. The kneaded product was extruded through a nozzle, and then cured at 40°C for 1 day to give a rod formulation with a diameter of 2.0 mm. The rod formulation was cut into a length of 7 mm to give Comparative Formulation 9.

### Comparative Example 10

PEG4000 (112 mg) and acetaminophen (8 mg) were mixed in a mortar to give a mixed powder. SILASTIC Q7-4750 Silicone A component (140 mg) and Silicone B component (140 mg) manufactured by Dow Corning were kneaded together with a twin roll. After the above silicones were kneaded, all of the above-obtained mixed powder was quickly added thereto, and the mixture was kneaded. The kneaded product was extruded through a nozzle, and then cured at 40°C for 1 day to give a rod formulation with a diameter of 2.0 mm. The rod formulation was cut into a length of 7 mm to give Comparative Formulation 10.

### Test Example 11

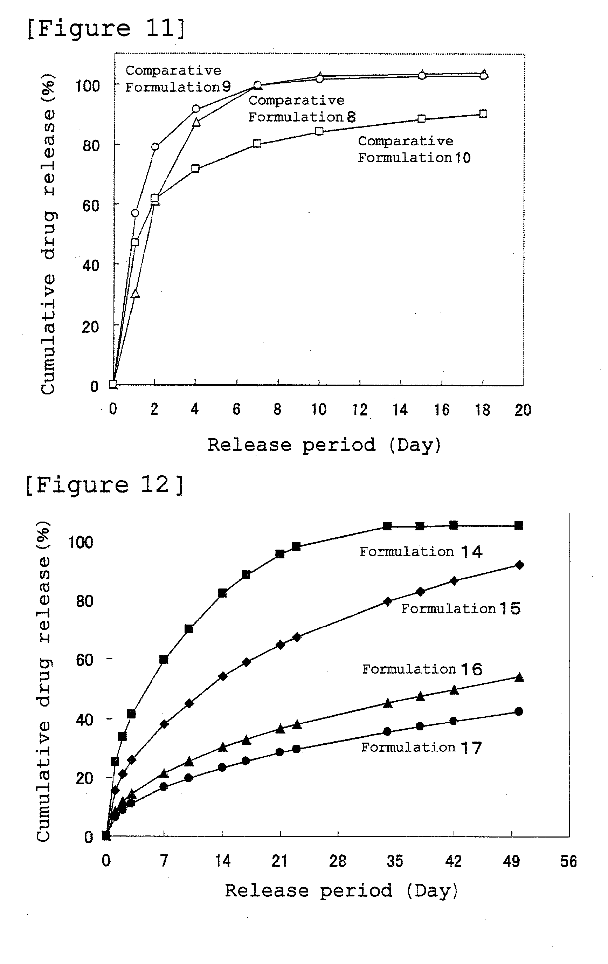

Each of the formulations obtained in Comparative Examples 8 to 10 (see, Table 11) was tested in the same manner as Test Example 5 to determine the cumulative percentage of drug release of acetaminophen (AA). The results are shown in Figure 11.

Comparative Formulations 8 to 10 do not contain the additive agents of the present invention. Instead, as an additive agent, Comparative Formulation 8 contains merely NaCl (which is a general water-soluble additive agent), Comparative Formulation 9 contains water-soluble HPC (which is disclosed in Patent Reference 4), and Comparative Formulation 10 contains PEG4000 (which is disclosed as a water-soluble additive agent in Patent Reference 4 and WO 00/15199). All of the formulations showed a markedly fast release-rate characterized by an initial burst, without a long-term sustained release.

In contrast, the present formulation attained an excellent sustained-release of acetaminophen for a long period as illustrated above.

**[Table 11]**

| | Dosage form | Drug | Concentration of drug (wt%) | water-soluble additive agents | Results cr notes | |
|---|---|---|---|---|---|---|
| Comparative Formulation 8 | Rod | AA | 2 | NaCl 28 wt% | Mater-soluble additive agent | In case that the formulation contains only water-soluble additive agent without any hardly water-soluble solid substance, poor sustained-release with initial burst is observed. |
| Comparative Formulation 9 | Rod | AA | 2 | HPC (containing 53.4 % to 77.5 % of hydroxypropoxyl group) 28 wt% | Additive agent disclosed in Patent Reference 4 | |
| Comparative Formulation 10 | Rod | AA | 2 | PEG4000 28 wt% | Additive agent disclosed in Patent Reference 4 and WO 00/15199 | |

### Example 14

L-HPC (170 mg) and acetaminophen (100 mg) were mixed in a mortar to give a mixed powder. SILASTIC Q7-4750 Silicone A component (365 mg) and Silicone B component (365 mg) manufactured by Dow Corning were kneaded together with a twin roll. After the above silicones were kneaded, all of the above-obtained mixed powder was quickly added thereto, and the mixture was kneaded. The kneaded product was extruded through a nozzle, and then cured at 40°C for 1 day to give a rod formulation with a diameter of 0.6 mm. The rod formulation was cut into a length of 10 mm to give Formulation 14.

### Example 15

L-HPC (170 mg) and acetaminophen (100 mg) were mixed in a mortar to give a mixed powder. SILASTIC Q7-4750 Silicone A component (365 mg) and Silicone B component (365 mg) manufactured by Dow Corning were kneaded together with a twin roll. After the above silicones were kneaded, all of the above-obtained mixed powder was quickly added thereto, and the mixture was kneaded. The kneaded product was extruded through a nozzle, and then cured at 40°C for 1 day to give a rod formulation with a diameter of 1.0 mm. The rod formulation was cut into a length of 10 mm to give Formulation 15.

### Example 16

L-HPC (170 mg) and acetaminophen (100 mg) were mixed in a mortar to give a mixed powder. SILASTIC Q7-4750 Silicone A component (365 mg) and Silicone B component (365 mg) manufactured by Dow Corning were kneaded together with a twin roll. After the above silicones were kneaded, all of the above-obtained mixed powder was quickly added thereto, and the mixture was kneaded. The kneaded product was extruded through a nozzle, and then cured at 40°C for 1 day to give a rod formulation with a diameter of 2.0 mm. The rod formulation was cut into a length of 10 mm to give Formulation 16.

### Example 17

L-HPC (170 mg) and acetaminophen (100 mg) were mixed in a mortar to give a mixed powder. SILASTIC Q7-4750 Silicone A component (365 mg) and Silicone B component (365 mg) manufactured by Dow Corning were kneaded together with a twin roll. After the above silicones were kneaded, all of the above-obtained mixed powder was quickly added thereto, and the mixture was kneaded. The kneaded product was extruded through a nozzle, and then cured at 40°C for 1 day to give a rod formulation with a diameter of 2.5 mm. The rod formulation was cut into a length of 10 mm to give Formulation 17.

### Test Example 12

Each of the formulations obtained in Examples 14 to 17 (see, Table 12) was put into PBS (1 mL), kept at 37°C, measured the acetaminophen (AA) released from the formulation with a high performance liquid chromatograph (UFLC, manufactured by Shimadzu Corporation), and the value was calculated to determine the cumulative percentage of drug release. The results are shown in Figure 12. Each of the formulations obtained in Examples 14 to 17 comprises the same components but has a different diameter, as shown in Table 12. All of the formulations (which vary in diameter) attained an excellent sustained-release lasting for 5 weeks or more. In addition, the results show that formulations with a larger diameter tend to prolong the period of releasing the drug. A sustained-release of drug is achieved on the basis of a mechanism wherein water slowly flows in from the formuiation's surface to internal part. The variation in diameter brings about a change in the distance between the surface and deepest part of the formulation, and thus the time required for water to reach to the deepest part of the formulation changes and the period of sustained-release changes.

**[Table 12]**

| | Dosage form | Drug | Concentration of drug (wt%) | Concentration of L-HPC (wt%) | Other additive agents | Diameter (mm) |
|---|---|---|---|---|---|---|
| Formulation 14 | Rod | AA | 10 | 17 | None | 0.6 |
| Formulation 15 | Rod | AA | 10 | 17 | None | 1.0 |
| Formulation 16 | Rod | AA | 10 | 17 | None | 2.0 |
| Formulation 17 | Rod | AA | 10 | 17 | None | 2.5 |

### Example 18

Crystalline sodium chloride was milled in a mortar to adjust the particle diameter thereof to 100 µm or less. To the above-obtained sodium chloride (30 mg) were added L-HPC (70 mg) and then acetaminophen (100 mg), and the three kinds of powder were mixed all together in the mortar to give a mixed powder. SILASTIC Q7-4750 Silicone A component (400 mg) and Silicone B component (400 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.8 mm. The sheet formulation was cut into a size of 5 mm x 7 mm to give Formulation 18.

### Example 19

Glycine was milled in a mortar to adjust the particle diameter thereof to 100 µm or less. To the above-obtained glycine (30 mg) were added L-HPC (70 mg) and then acetaminophen (100 mg), and the three kinds of powder were mixed all together in the mortar to give a mixed powder. SILASTIC Q7-4750 Silicone A component (400 mg) and Silicone B component (400 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.8 mm. The sheet formulation was cut into a size of 5 mm x 7 mm to give Formulation 19.

### Example 20

To glucose (30 mg) were added L-HPC (70 mg) and then acetaminophen (100 mg), and the three kinds of powder were mixed all together in a mortar to give a mixed powder. SILASTIC Q7-4750 Silicone A component (400 mg) and Silicone B component (400 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.8 mm. The sheet formulation was cut into a size of 5 mm x 7 mm to give Formulation 20.

### Example 21

To mannitol (30 mg) were added L-HPC (70 mg) and then acetaminophen (100 mg), and the three kinds of powder were mixed all together in a mortar to give a mixed powder. SILASTIC Q7-4750 Silicone A component (400 mg) and Silicone B component (400 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.8 mm. The sheet formulation was cut into a size of 5 mm x 7 mm to give Formulation 21.

### Example 22

To lactose (30 mg) were added L-HPC (70 mg) and then acetaminophen (100 mg), and the three kinds of powder were mixed all together in a mortar to give a mixed powder. SILASTIC Q7-4750 Silicone A component (400 mg) and Silicone B component (400 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.8 mm. The sheet formulation was cut into a size of 5 mm x 7 mm to give Formulation 22.

### Test Example 13

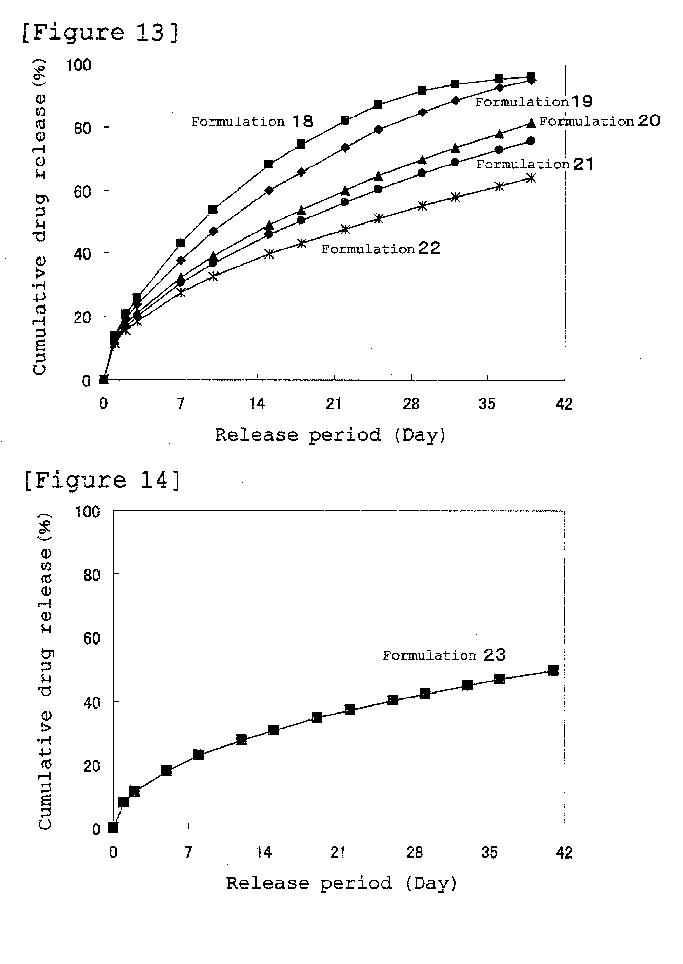

Each of the formulations obtained in Examples 18 to 22 (see, Table 13) was tested in the same manner as Test Example 12 to determine the cumulative percentage of drug release of acetaminophen (AA). The results are shown in Figure 13. Each of the formulations contains a different water-soluble additive agent, but all of the formulations attained an excellent sustained-release lasting for 5 weeks or more.

**[Table 13]**

| | Dosage form | Drug | Concentration of drug (wt%) | Concentration of L-HPC (wt%) | Other additive agents |
|---|---|---|---|---|---|
| Formulation 18 | Sheet | AA | 10 | 7 | Sodium chloride 3 wt% |
| Formulation 19 | Sheet | AA | 10 | 7 | Glycine 3 wt% |
| Formulation 20 | Sheet | AA | 10 | 7 | Glucose 3 wt% |
| Formulation 21 | Sheet | AA | 10 | 7 | Mannitol 3 wt% |
| Formulation 22 | Sheet | AA | 10 | 7 | Lactose 3 wt% |

### Example 23

Crystalline sodium chloride was milled in a mortar to adjust the particle diameter thereof to 100 µm or less. To the above-obtained sodium chloride (30 mg) were added crospovidone (170 mg) and then droxidopa (100 mg), and the three kinds of powder were mixed all together in the mortar to give a mixed powder. SILASTIC Q7-4750 Silicone A component (350 mg) and Silicone B component (350 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.8 mm. The sheet formulation was cut into a size of 5 mm x 7 mm to give Formulation 23.

### Test Example 14

Formulation 23 obtained in Example 23 (see, Table 14) was put into PBS (1 mL), kept at 5°C, measured the droxidopa released from the formulation with a high performance liquid chromatograph (UFLC, manufactured by Shimadzu Corporation), and the value was calculated to determine the cumulative percentage of drug release. As shown in Figure 14, the formulation attained an excellent sustained-release lasting for 6 weeks. In addition, droxidopa is thermally unstable in solution state and thus the release test was carried out at 5°C, but the thermal stability thereof in solution state may be improved by adding, for example, a stabilizing agent.

**[Table 14]**

| | Dosage form | Drug | Concentration of drug (wt%) | Concentration of crospovidone (wt%) | Other additive agents |
|---|---|---|---|---|---|
| Formulation 23 | Sheet | Droxidopa | 10 | 17 | sodium chloride 3 wt% |

### Example 24

L-HPC (70 mg) and tranexamic acid (100 mg) were mixed in a mortar to give a mixed powder. SILASTIC Q7-4750 Silicone A component (415 mg) and Silicone B component (415 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.8 mm. The sheet formulation was cut into a size of 5 mm x 7 mm to give Formulation 24.

### Test Example 15

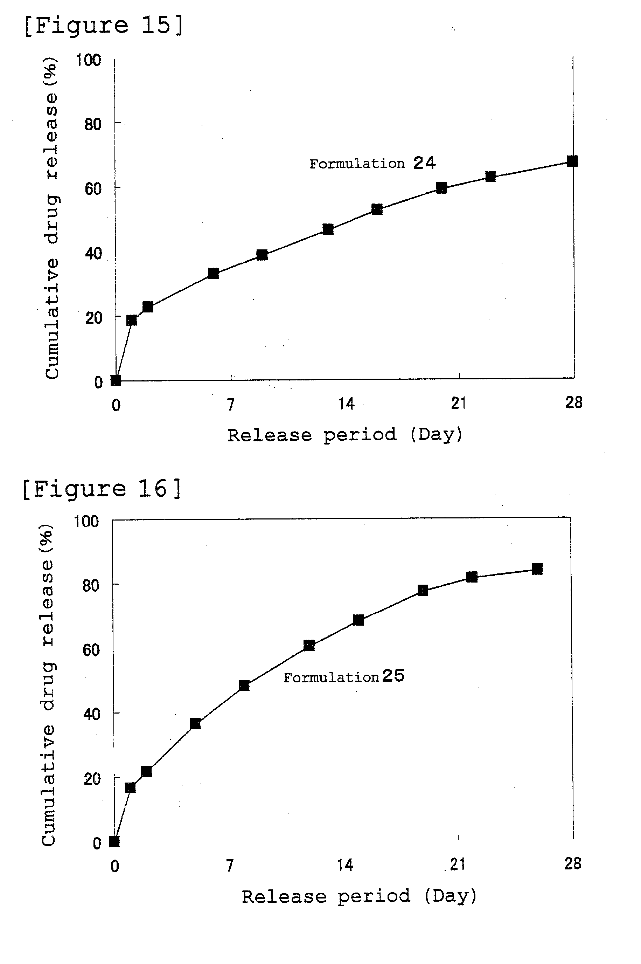

Formulation 24 obtained in Example 24 (see, Table 15) was put into PBS (1 mL), kept at 37°C, measured the tranexamic acid released from the formulation with a high performance liquid chromatograph (UFLC, manufactured by Shimadzu Corporation), and the value was calculated to determine the cumulative percentage of drug release. As shown in Figure 15, the formulation attained an excellent sustained-release lasting for 4 weeks.

**[Table 15]**

| | Dosage form | Drug | Concentration of drug (wt%) | Concentration of L-HPC (wt%) | Other additive agents |
|---|---|---|---|---|---|
| Formulation 24 | Sheet | Tranexamic acid | 10 | 7 | None |

### Example 25

L-HPC (170 mg) and L-phenylalanine (100 mg) were mixed in a mortar to give a mixed powder. SILASTIC Q7-4750 Silicone A component (365 mg) and Silicone B component (365 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.8 mm. The sheet formulation was cut into a size of 5 mm x 7 mm to give Formulation 25.

### Test Example 16

Formulation 25 obtained in Example 25 (see, Table 16) was put into PBS (1 mL), kept at 37°C, measured the L-phenylalanine released from the formulation with a high performance liquid chromatograph (UFLC, manufactured by Shimadzu Corporation), and the value was calculated to determine the cumulative percentage of drug release. As shown in Figure 16, the formulation attained an excellent sustained-release lasting for 4 weeks.

**[Table 16]**

| | Dosage form | Drug | Concentration of drug (wt%) | Concentration of L-HPC (wt%) | Other additive agents |
|---|---|---|---|---|---|
| Formulation 27 | Sheet | L-phenylalanine | 10 | 17 | None |

### Example 26

L-HPC (70 mg) and metformin hydrochloride (100 mg) were mixed in a mortar to give a mixed powder. SILASTIC Q7-4750 Silicone A component (415 mg) and Silicone B component (415 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.8 mm. The sheet formulation was cut into a size of 5 mm x 7 mm to give Formulation 26.

### Test Example 17

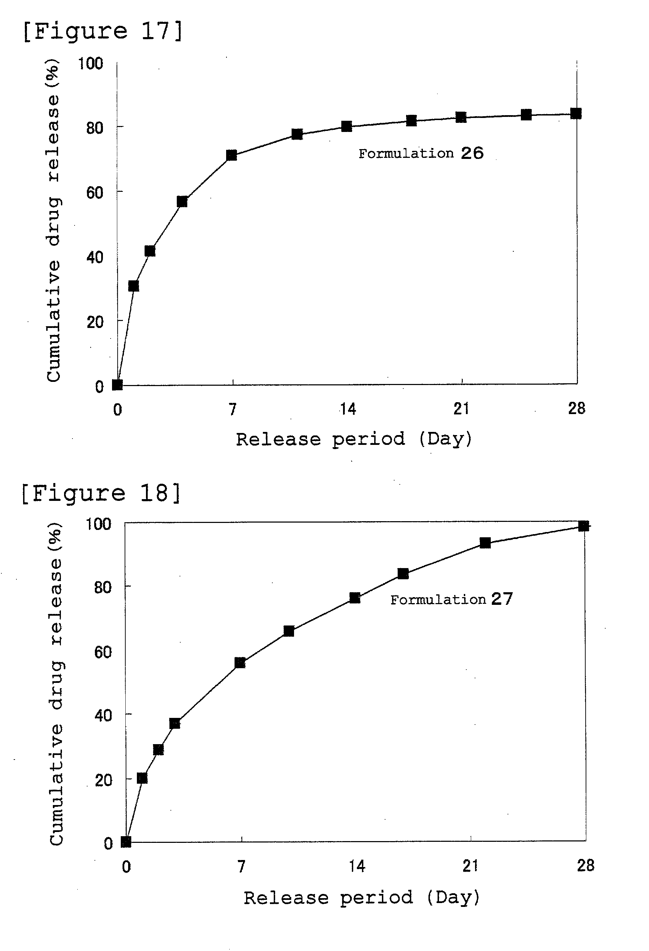

Formulation 26 obtained in Example 26 (see, Table 17) was put into PBS (1 mL), kept at 37°C, measured the metformin hydrochloride released from the formulation with a high performance liquid chromatograph (UFLC, manufactured by Shimadzu Corporation), and the value was calculated to determine the cumulative percentage of drug release. As shown in Figure 17, the formulation attained an excellent sustained-release lasting for 2 weeks.

**[Table 17]**

| | Dosage form | Drug | Concentration of drug (wt%) | Concentration of L-HPC (wt%) | Other additive agents |
|---|---|---|---|---|---|
| Formulation 26 | Sheet | Metformin hydrochloride | 10 | 7 | None |

### Example 27

L-HPC (170 mg) and zonisamide (100 mg) were mixed in a mortar to give a mixed powder. SILASTIC Q7-4750 Silicone A component (365 mg) and Silicone B component (365 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.8 mm. The sheet formulation was cut into a size of 5 mm x 7 mm to give Formulation 27.

### Test Example 18

Formulation 27 obtained in Example 27 (see, Table 18) was put into PBS (1 mL), kept at 37°C, measured the zonisamide released from the formulation with a high performance liquid chromatograph (UFLC, manufactured by Shimadzu Corporation), and the value was calculated to determine the cumulative percentage of drug release. As shown in Figure 18, the formulation attained an excellent sustained-release lasting for 4 weeks.

**[Table 18]**

| | Dosage form | Drug | Concentration of drug (wt%) | Concentration Of L-HPC (wt%) | Other additive agents |
|---|---|---|---|---|---|
| Formulation 27 | Sheet | Zonisamide | 10 | 17 | None |

### Example 28

L-HBC (170 mg) and acetaminophen (100 mg) were mixed in a mortar to give a mixed powder. SILASTIC Q7-9750 Silicone A component (365 mg) and Silicone B component (365 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.8 mm. The sheet formulation was cut into a size of 5 mm x 7 mm to give Formulation 28.

### Example 29

Partly pregelatinized starch (170 mg) and acetaminophen (100 mg) were mixed in a mortar to give a mixed powder. SILASTIC Q7-4750 Silicone A component (365 mg) and Silicone B component (365 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.8 mm. The sheet formulation was cut into a size of 5 mm x 7 mm to give Formulation 29.

### Example 30

Crospovidone (70 mg) and acetaminophen (100 mg) were mixed in a mortar to give a mixed powder. SILASTIC Q7-4750 Silicone A component (415 mg) and Silicone B component (415 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.8 mm. The sheet formulation was cut into a size of 5 mm x 7 mm to give Formulation 30.

### Example 31

Croscarmellose sodium (70 mg) and acetaminophen (100 mg) were mixed in a mortar to give a mixed powder. SILASTIC Q7-4750 Silicone A component (415 mg) and Silicone B component (415 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.8 mm. The sheet formulation was cut into a size of 5 mm x 7 mm to give Formulation 31.

### Test Example 19

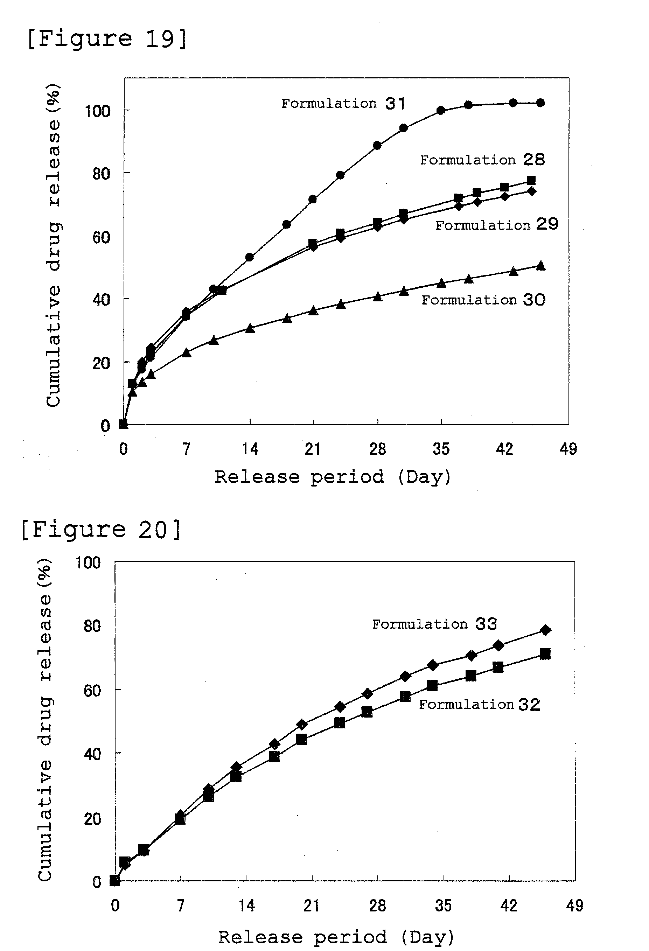

Each of the formulations obtained in Examples 28 to 31 (see, Table 19) was tested in the same manner as Test Example 12 to determine the cumulative percentage of drug release of acetaminophen (AA). The results are shown in Figure 19. Each of the formulations contains a different hardly water-soluble substance, but all of the formulations attained an excellent sustained-release lasting for 5 weeks or more.

**[Table 19]**

| | Dosage form | Drug | Concentration of drug (wt%) | Hardly water-soluble substance | Other additive agents |
|---|---|---|---|---|---|
| Formulation 28 | Sheet | AA | 10 | L-HPC 17 wt% | None |
| Formulation 29 | Sheet | AA | 10 | Partly pregelatinized starch 17 wt% | None |
| Formulation 30 | Sheet | AA | 10 | Crospovidone 7 wt% | None |
| Formulation 31 | Sheet | AA | 10 | CrosCMC-Na 7 wt% | None |

### Example 32

Crystalline sodium chloride was milled in a mortar to adjust the particle diameter thereof to 100 µm or less. To the above-obtained sodium chloride (30 mg) were added sodium cholate (85 mg), cholesterol (85 mg) and then zonisamide (100 mg), and the four kinds of powder were mixed all together in the mortar to give a mixed powder. STLASTIC Q7-4750 Silicone A component (350 mg) and Silicone B component (350 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.8 mm. The sheet formulation was cut into a size of 5 mm x 7 mm to give Formulation 32.

### Example 33

Crystalline sodium chloride was milled in a mortar to adjust the particle diameter thereof to 100 µm or less. To the above-obtained sodium chloride (30 mg) were added sodium glycocholate (85 mg), cholesterol (85 mg) and then zonisamide (100 mg), and the four kinds of powder were mixed all together in the mortar to give a mixed powder. SILASTIC Q7-4750 Silicone A component (350 mg) and Silicone B component (350 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.8 mm. The sheet formulation was cut into a size of 5 mm x 7 mm to give Formulation 33.

### Test Example 20

Each of the formulations obtained in Examples 32 and 33 (see, Table 20) was tested in the same manner as Test Example 18 to determine the cumulative percentage of drug release of zonisamide. The results are shown in Figure 20. Each of the formulation contains cholesterol as the hardly water-soluble solid substance, and "cholate and NaCl" or "glycocholate and NaCl" as the water-soluble additive agent, but all of the formulations attained an excellent sustained-release lasting for 6 weeks or more.

**[Table 20]**

| | Dosage form | Drug | Concentration of drug (wt%) | Concentration of cholesterol (wt%) | Other additive agents |
|---|---|---|---|---|---|
| Formulation 32 | Sheet | Zonisamide | 10 | 8.5 | Sodium cholate 8.5 wt% + NaCl 3 wt% |
| Formulation 33 | Sheet | Zonisamide | 10 | 8.5 | Sodium glycocholate 8.5 wt% + NaCl 3 wt% |

### Example 34

Crystalline sodium chloride was milled in a mortar to adjust the particle diameter thereof to 100 µm or less. To the above-obtained sodium chloride (15 mg) were added saccharin (185 mg) and then zonisamide (100 mg), and the three kinds of powder were mixed all together in the mortar to give a mixed powder. SILASTIC Q7-4750 Silicone A component (350 mg) and Silicone B component (350 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.8 mm. The sheet formulation was cut into a size of 5 mm x 7 mm to give Formulation 34.

### Example 35

Crystalline sodium chloride was milled in a mortar to adjust the particle diameter thereof to 100 µm or less. To the above-obtained sodium chloride (30 mg) were added myristic acid (70 mg) and then zonisamide (100 mg), and the three kinds of powder were mixed all together in the mortar to give a mixed powder. SILASTIC Q7-4750 Silicone A component (400 mg) and Silicone B component (400 mg) manufactured by Dow Corning were kneaded together with a twin roll. After the above silicones were kneaded, all of the above-obtained mixed powder was quickly added thereto, and the mixture was kneaded. Then, the kneaded product was rolled into a sheet shape with a twin roll and cured at 40°C for 1 day to give a sheet formulation with a thickness of 0.8 mm. The sheet formulation was cut into a size of 5 mm x 7 mm to give Formulation 35.

### Test Example 21

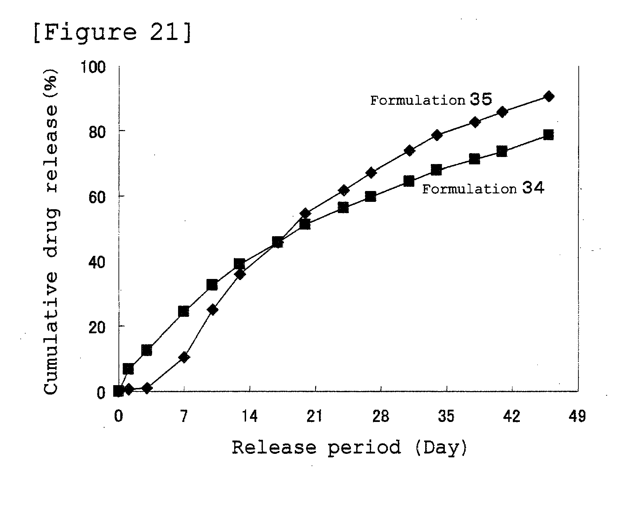

Each of the formulations obtained in Examples 34 and 35 (see, Table 21) was tested in the same manner as Test Example 18 to determine the cumulative percentage of drug release of zonisamide. The results are shown in Figure 21. Each of the formulations contains saccharin or myristic acid as the hardly water-soluble solid substance, and NaCl as the water-soluble additive agent, but all of the formulations attained an excellent sustained-release lasting for 6 weeks or more.

**[Table 21]**

| | Dosage form | Drug | Concentration of drug (wt%) | Hardly water-soluble substance | Other additive agents |
|---|---|---|---|---|---|
| Formulation 34 | Sheet | Zonisamide | 10 | Saccharin 18.5 wt% | NaCl 1.5 wt% |
| Formulation 35 | Sheet | Zonisamide | 10 | Myristic acid 7 wt% | NaCl 3 wt% |

## Claims

1. A parenteral solid formulation comprising a water-soluble drug and a hardly water-soluble pharmaceutically-acceptable solid substance, which comprises silicone as a carrier.

2. The parenteral solid formulation of claim 1 wherein the hardly water-soluble substance is low substituted hydroxypropylcellulose, partly pregelatinized starch, crospovidone, croscarmellose sodium, myristic acid, cholesterol and/or saccharin.

3. The parenteral solid formulation of claim 1 wherein the hardly water-soluble substance is low substituted hydroxypropylcellulose and/or cholesterol.

4. The parenteral solid formulation of any one of claims 1 to 3 wherein the hardly water-soluble substance is contained in 3 to 35 % by weight per the whole weight of the formulation.

5. The parenteral solid formulation of any one of claims 1 to 4 wherein the silicone is contained in 55 % or more by weight per the whole weight of the formulation.

6. The parenteral solid formulation of any one of claims 1 to 5 further comprising a water-soluble additive agent.

7. The parenteral solid formulation of claim 6 wherein the water-soluble additive agent is sodium chloride, glucose, mannitol, lactose, glycine, sodium cholate, sodium desoxycholate and/or sodium glycocholate.

8. The parenteral solid formulation of claim 6 wherein the water-soluble additive agent is sodium chloride and/or sodium desoxycholate.

9. The parenteral solid formulation of claim 8 wherein the hardly water-soluble substance is low substituted hydroxypropylcellulose, and the water-soluble additive agent is sodium chloride.

10. The parenteral solid formulation of claim 8 wherein the hardly water-soluble substance is cholesterol, and the water-soluble additive agent is sodium chloride and sodium desoxycholate.

11. The parenteral solid formulation of any one of claims 1 to 10 wherein the total weight of the water-soluble drug, the hardly water-soluble substance and the optional water-soluble additive agent is 10 to 40 % per the whole weight of the formulation, provided that the total weight of the water-soluble drug and the water-soluble additive agent is not more than 35 % per the whole weight of the formulation.

12. The parenteral solid formulation of any one of claims 1 to 11 comprising essentially a water-soluble drug, a pharmaceutically acceptable hardly water-soluble solid substance, and an optional water-soluble additive agent, and which comprises silicone as a carrier.

13. The parenteral solid formulation of any one of claims 1 to 12 which is a matrix formulation.

14. The parenteral solid formulation of any one of claims 1 to 13 which is an implantable formulation.
